# EUROPÄISCHE PATENTSCHRIFT

(11) **EP 1 747 035 B1**
(45) Veröffentlichungstag und Bekanntmachung des Hinweises auf die Patenterteilung: **17.08.2011**
(21) Anmeldenummer: 05744279.0
(22) Anmeldetag: 03.05.2005
(51) Int. Cl.: A61M 15/00, B05B 11/00

(54) **ZERSTÄUBER ZUM AUSBRINGEN VON FLÜSSIGKEITEN FÜR MEDIZINISCHE ZWECKE**
Spray for liquids for medical purposes.
ATOMISEUR POUR LA DISTRIBUTION DE LIQUIDES A DES FINS MEDICALES

(30) Priorität: 03.05.2004 DE 102004021789
(43) Veröffentlichungstag der Anmeldung: 31.01.2007
(73) Patentinhaber: Boehringer Ingelheim International GmbH, 55216 Ingelheim am Rhein (DE)
(72) Erfinder: JUNG, Andree, 55743 Idar-Oberstein (DE); SPALLEK, Michael, 55218 Ingelheim (DE)
(74) Vertreter: Hammann, Heinz
(86) Internationale Anmeldenummer: PCT/EP2005/004792
(87) Internationale Veröffentlichungsnummer: WO 2005/107837

(56) Entgegenhaltungen:
- EP-A- 0 520 571
- WO-A-97/12687
- US-A- 5 289 948
- US-A- 5 339 990
- US-A1- 2002 129 812

## Beschreibung

Die Erfindung betrifft einen Zerstäuber zum Ausbringen von Flüssigkeiten für medizinische Zwecke, beispielsweise pharmazeutischen Wirkstoffformulierungen, aus mindestens einem in den Zerstäuber einsetzbaren Behälter für die Flüssigkeit (Kartusche).

Die bekannten Zerstäuber umfassen als wesentliche Baugruppen einen in dem Gehäuse angeordneten Kartuschenhalter, ein an dem Gehäuse ausgebildetes Mundstück, in welchem eine Zerstäubungsvorrichtung angeordnet ist, und ein Verbindungsrohrsystem, welches die Zerstäubungsvorrichtung und den Kartuschenhalter verbindet, wobei die Zerstäubungsvorrichtung mindestens einen Düsenkörper und das Verbindungsrohrsystem mindestens einen Kanal mit einem darin verschiebbar gelagerten Hohlkolben aufweist, wobei der Hohlkolben einen Ventilkörper umfaßt.

Die in den Zerstäubern eingesetzten Hohlkolben umfassen einen Ventilkörper. Der Ventilkörper ermöglicht bei einer Abwärtsbewegung des Hohlkolbens in einer geöffneten Stellung ein Einströmen der Formulierung oder bereits gemischten Formulierungen in den in Strömungsrichtung hinter dem Hohlkolben liegenden Abschnitt des Kanals. Bei einer Aufwärtsbewegung des Hohlkolbens verschließt der Ventilkörper den Hohlkolben und bei weiterer Aufwärtsbewegung wird die in Strömungsrichtung hinter dem Hohlkolben befindliche Formulierung oder das Gemisch auf den gewünschten Druck verdichtet.

Derartige Zerstäuber werden für die Applikation von inhalativ wirksamen flüssigen Arzneistoffformulierungen benötigt, wobei die flüssigen Formulierungen hohen Qualitätsstandards genügen müssen. Um eine gute Wirkstoffverteilung der Wirksubstanzen in der Lunge zu erhalten, bietet sich die Applikation einer flüssigen, auf Treibgase verzichtenden Formulierung mittels dafür geeigneter Zerstäuber oder Inhalatoren an. Besonders geeignet sind solche Zerstäuber, die eine kleine Menge einer flüssigen Formulierung in der therapeutisch notwendigen Dosierung binnen weniger Sekunden in ein therapeutisch-inhalativ geeignetes Aerosol vernebeln können. Dabei sollte die Vernebelungsdauer zeitlich optimiert sein, um eine optimale Lungendeposition zu garantieren. Im Rahmen der vorliegenden Erfindung sind solche Vernebler bevorzugt, bei denen bereits eine Menge von weniger als 100 Mikroliter, bevorzugt weniger als 50 Mikroliter, ganz bevorzugt weniger als 20 Mikroliter Wirkstofflösung mit einem Hub oder wenigen Hüben zu einem Aerosol mit einer durchschnittlichen Teilchengröße von weniger als 20 Mikrometern, bevorzugt weniger als 10 Mikrometern, vernebelt werden.

Eine derartige Vorrichtung zur treibgasfreien Verabreichung einer dosierten Menge eines flüssigen Arzneimittels zur inhalativen Anwendung wird beispielsweise in der internationalen Patentanmeldung WO 91/14468 "Atomizing Device and Methods" oder auch in der WO 97/12687 ausführlich beschrieben. In einem solchen Zerstäuber wird eine Arzneimittellösung mittels hohen Drucks von bis zu 500 bar in ein lungengängiges Aerosol überführt.

In den bekannten Zerstäubern werden die Wirkstoffformulierungen als Lösungen in einem Reservoir gelagert. Dabei ist es notwendig, dass diese verwendeten Lösungen eine ausreichende Lagerstabilität aufweisen und gleichzeitig so beschaffen sind, dass sie dem medizinischen Zweck entsprechend möglichst ohne weitere Manipulation direkt appliziert werden können. Ferner dürfen sie keine Bestandteile aufweisen, die mit dem Zerstäuber wechselwirken können, so dass weder der Zerstäuber noch die Lösung oder das erzeugte Aerosol Schaden nimmt.

Zur Vernebelung der Lösung wird eine spezielle Düse verwendet, wie sie beispielsweise die WO 94/07607 oder die WO 99/16530 oder US-A-2002-129812 beschreibt,

Eine weiterentwickelte Ausführungsform des bevorzugten Inhalators wird auch in der WO 97/12687 offenbart.

Aufgrund seiner zylinderähnlichen Form und einer handlichen Größe von weniger als 9 bis 15 cm in der Länge und 2 bis 4 cm in der Breite können die bekannten Zerstäuber jederzeit von dem Patienten mitgeführt werden. Der Zerstäuber versprüht ein definiertes Volumen der Arzneimittelformulierung unter Anwendung hoher Drücke durch kleine Düsen, so dass inhalierbare Aerosole entstehen.

Die EP 0918570 beschreibt einen Zerstäuber für Nasensprays, der als Kernelemente einen federbetriebenen Kolben und eine Düseneinrichtung enthält. Zwischen dem Kolben und der Düse kann ein Behälter eingelegt werden, der bodenseitig einen Stempel aufweist und kopfseitig über eine Dichtung verschlossen ist. Diese Dichtung des Behälters wird vor dem ersten Gebrauch durch Bewegen der externen, in dem Zerstäuber integrierten Düse durch die Dichtung hindurch geöffnet.

Die beschriebenen Zerstäuber sind jedoch primär für die Abgabe von stabilen Lösungsformulierungen geeignet, d.h. für Formulierungsbestandteile, die stabil miteinander gemischt und längere Zeit gelagert werden können. So ist es beispielsweise bei Kombinationspräparaten, die mindestens zwei Wirkstoffe beinhalten, Voraussetzung, dass die zu verabreichenden Wirkstoffe z.B. bei dem selben pH-Wert in dem selben Lösungsmittel stabil sind.

Aus dem Stand der Technik sind außerdem verschiedene Prinzipien von Geräten zur Verabreichung von Medikamenten bekannt, die vor ihrer Verabreichung getrennt aufbewahrt werden, aber sodann als Mischung dem Patienten zur Verfügung stehen.

Aus WO2004/011068 ist ein elektronisches System zur Verabreichung von Medikamentenmischungen bekannt.

WO2004/01107 offenbart ein System zur Verabreichung von Medikamenten, wobei die beiden Medikamentenbehälter von unterschiedlicher Bauart sind.

Die W02004/011067 und 011070 offenbaren ebenfalls Systeme zur Verabreichung von Medikamentenmischungen, wobei in der 01170 die Verabreichung von mehreren co-formulierten Medikamenten mit einem Medikament, welches sich nicht zur Co-Formulierung eignet, beansprucht wird.

W02004/011067 beansprucht ein einfaches System zur Verabreichung von Medikamenten, die in getrennten Behältnissen aufbewahrt werden, jedoch als Mischung dem Patienten verabreicht werden können.

Der Erfindung lag nunmehr die Aufgabe zugrunde, einen Zerstäuber bereitzustellen, der es ermöglicht, auch Formulierungen zu lagern und zu verabreichen, die nur unter vergleichsweise unterschiedlichen Bedingungen, wie z.B. pH-Wert, Lösungsmittel oder Hilfsstoffe, stabil gelagert werden können.

Darüber hinaus sollte eine Kartusche zur Lagerung der Formulierungen bereitgestellt werden.

Eine weitere Teilaufgabe bestand in der Bereitstellung eines Systems bestehend aus einem Zerstäuber und einer Kartusche.

Die Aufgabe wird erfindungsgemäß zunächst mit einem Zerstäuber, wie im Anspruch 1 definiert wird, gelöst.

Als besonders günstig hat es sich erwiesen, den ersten Düsenkörper mit zwei Düsenauslässen und den zweiten Düsenkörper ebenfalls mit zwei Düsenauslässen zu versehen, wobei die zwei Düsenauslässe eines Düsenkörpers zueinander geneigt sind. Hierbei werden zwei Flüssigkeiten zeitlich parallel zerstäubt, so dass zwei Aerosol-Wolken entstehen, die sich gegenseitig durchdringen. Anstelle von zwei getrennten Düsenauslässen pro Düsenkopf sind prinzipiell noch weitere Düsenauslässe möglich.

Mit Hilfe mehrerer getrennter Düsenkörper, die jeweils zwei oder mehrere Düsenauslässe aufweisen, lassen sich darüber hinaus unterschiedliche Partikelgrößen aus den unterschiedlichen Formulierungen gleichzeitig erzeugen. So kann beispielsweise eine größere Partikelgröße einer ersten Formulierung für die Behandlung der oberen Atemwege verabreicht werden und gleichzeitig eine kleinere lungengängige Partikelgröße der zweiten Formulierung für die Behandlung der unteren Atemwege.

In allen Ausführungsformen können die geneigten Düsenauslässe derart ausgerichtet sein, dass sie Strahlrichtungen der Flüssigkeiten unter einem Aufprallwinkel von bevorzugt 20° bis 160°, besonders bevorzugt 60° bis 150° und ganz besonders bevorzugt 80° bis 100° erzeugen. Durch den Aufprallwinkel wird maßgeblich der Impaktionsabstand bestimmt, das heißt der Abstand von den Düsenauslässen, in welchem sich beide Flüssigkeiten treffen. Dieser Impaktionsabstand darf nicht zu groß gewählt werden, da ansonsten keine ausreichende Zerstäubung stattfindet.

Die Düsenauslässe auf einem Düsenkörper sind vorzugsweise in einer Entfernung von 10 µm bis 200 µm, bevorzugt von 10 µm bis 100 µm, besonders bevorzugt 30 µm bis 70 µm, am stärksten bevorzugt 50 µm angeordnet.

Vorteilhafterweise ist der Ventilkörper an dem in Strömungsrichtung hinteren Ende des Hohlkolbens angeordnet. Hierdurch wird ein Rückströmen der Formulierung oder der gemischten Formulierungen in den Hohlkolben verhindert.

Günstigerweise ist der mindestens eine Düsenkörper ortsfest an dem mindestens einen Kanal befestigt.

Vorzugsweise ist der Kartuschenhalter beweglich gelagert.

Zur Speicherung einer vorbestimmten Energiemenge kann in dem Gehäuse ein Spannelement angeordnet sein. Als Spannelement kann beispielsweise eine in ihrer axialen Richtung spannbare Schraubenfeder dienen. Bei Betätigung einer Auslösetaste stellt das vorgespannte Spannelement die Energiemenge bereit und verschiebt den Kartuschenhalter in axialer Richtung des Gehäuses, d.h. in Richtung der Zerstäubungsvorrichtung. Die Eigenschaften des Spannelementes bestimmen die auf die Formulierungen einbringbare Energiemenge und somit wesentlich den erzielbaren Fluiddruck der Formulierungen. Als Fluiddruck werden 100 bar bis 700 bar, vorzugsweise 200 bar bis 500 bar, vorgesehen.

Die erste Teilaufgabe wird mit einer Kartusche gelöst, welche eine erste und eine zweite Kartuschenkammer aufweist, wobei eine erste Flüssigkeit in einer ersten Kartuschenkammer und eine zweite Flüssigkeit in einer räumlich von der ersten Kartuschenkammer getrennten zweiten Kartuschenkammer gelagert ist. Hierbei befinden sich beide Flüssigkeiten in einer Kartusche, wodurch die Handhabbarkeit beim Einsetzen der Kartusche vereinfacht wird.

Vorzugsweise weist die Kartusche eine formstabile Umfangs-, Deckel- und Bodenwand auf.

Die weitere Teilaufgabe wurde durch ein System gelöst, bei dem eine erste Flüssigkeit in einer ersten Kartusche oder Kartuschenkammer und eine zweite Flüssigkeit in einer räumlich von der ersten Kartusche oder Kartuschenkammer getrennten zweiten Kartusche oder Kartuschenkammer bevorratet ist und bei dem in die erste Kartusche oder Kartuschenkammer ein erster Hohlkolben und in die zweite Kartusche ein zweiter Hohlkolben oder in die erste Kartusche oder erste Kartuschenkammer ein mit dem Hohlkolben in Verbindung stehender erster Teilkolben und in die zweite Kartusche oder zweite Kartuschenkammer ein zweiter Teilkolben einschiebbar ist.

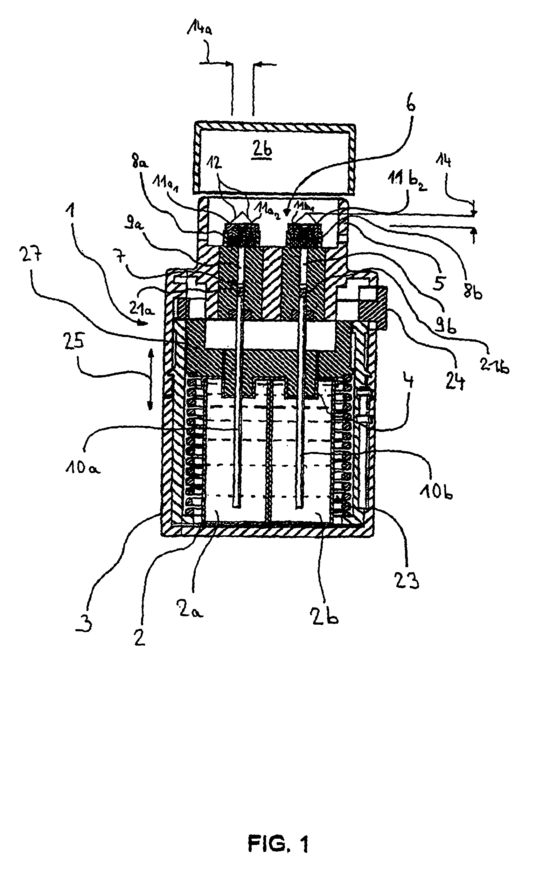

Zum besseren Verständnis wird im Folgenden die Erfindung anhand der Figuren näher erläutert. Lediglich Figur 1 zeigt die Erfindung. Dabei zeigen die:
- **Fig.1:**: einen Längsschnitt durch den Zerstäuber mit zwei Düsenkörpern und zwei parallel verlaufenden Kanälen mit darin geführten Hohlkolben nach einer ersten Ausführungsform mit zwei Kartuschenkammern in einer Kartusche;
- **Fig. 2:**: eine Ansicht gemäß Fig. 1 mit einem Düsenkörper und zwei parallel verlaufenden Kanälen mit darin geführten Hohlkolben ;
- **Fig. 3:**: eine Ansicht gemäß Fig. 1 mit einem Düsenkörper und einem Mischkanal, in welchem der erste und zweite Kanal zusammengeführt sind;
- **Fig. 4:**: eine Teilansicht gemäß Fig. 1 mit einem Düsenkörper und darin angeordneter Plenumkammer, in welcher der erste und zweite Kanal zusammengeführt ist;
- **Fig. 5:**: eine Ansicht gemäß Fig. 1 mit einem Düsenkörper und einem in Teilkolben aufgezweigten Hohlkolben; und die
- **Fig. 6:**: einen Längsschnitt durch eine Kartusche mit einer ersten und zweiten Kartuschenkammer.

Nachfolgend sind gleiche Teile mit denselben Bezugszeichen versehen.

Die Fig. 1 zeigt einen Längsschnitt durch den Zerstäuber 1 mit einem ersten Düsenkörper 8a und einem zweiten Düsenkörper 8b und zwei parallel verlaufenden Kanälen 9a, 9b mit darin geführten Hohlkolben 10a, 10b nach einer ersten Ausführungsform. Die Kanäle 9a, 9b und die Hohlkolben 10a, 10b bilden zusammen das Verbindungsrohrsystem 7

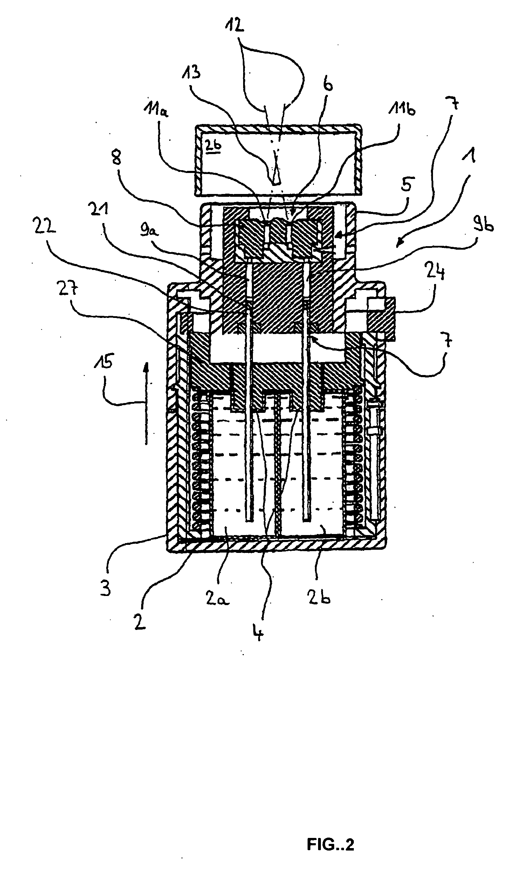

Der Zerstäuber umfasst ein zweigeteiltes und gegeneinander verdrehbares Gehäuse 3, über welches ein Spannelement 23 gespannt werden kann. Bei Betätigung einer Auslösetaste 24 wird ein topfförmiger Schubteller 27 freigegeben und durch die Federkraft des Spannelementes 23 in Strömungsrichtung 15 (siehe Fig. 2) vorgeschoben. Dabei werden ebenfalls die beiden an dem Schubteller 27 befestigten und diesen durchlaufenden Hohlkolben 10a, 10b zusammen mit dem Schubteller 27 vorgeschoben und verdichten dabei die zuvor in den Kanälen 9a, 9b angesaugten Flüssigkeiten, die anschließend aus dem ersten Düsenkörper 8a und dem zweiten Düsenkörper 8b austreten. Die Bildung von zwei separaten Aerosolwolken erfolgt über jeweils in dem Düsenkörper 8a, 8b angeordnete Düsenauslässe 11a₁, 11 a₂, 11b₁, 11b_{2,} das heißt insgesamt vier Düsenauslässe.

Für einen erneuten Gebrauch wird das Spannelement 23 wieder gespannt, wodurch der Schubteller 27 und die Hohlkolben 10a, 10b in Abwärtsbewegung versetzt werden. Durch die Abwärtsbewegung der Hohlkolben 10a, 10b wird aus den Kartuschenkammern 2a, 2b die jeweilige Formulierung in den jeweiligen ersten Kanal 9a oder zweiten Kanal 9b aufgezogen. In dieser Abwärtsbewegung sind die beiden Ventilkörper 21 a, 21 b in einer Durchlassstellung.

Bei Betätigung der Auslösetaste 24 werden die Hohlkolben 10a, 10b in axialer Richtung 25 des Gehäuses 3 verschoben, das heißt in eine Aufwärtsbewegung versetzt. Hierbei schließt der jeweilige Ventilkörper 21 a, 21 b selbsttätig den ersten Hohlkolben 10a oder den zweiten Hohlkolben 10b ab und ermöglicht dadurch die Verdichtung. Die Ventilkörper 21 a, 21 b befinden sich an dem hinteren Ende 22 (s.

Fig. 2) des jeweiligen Hohlkolbens 10a, 10b.

Während der Aufwärtsbewegung des ersten Hohlkolbens 10a und des zweiten Hohlkolbens 10b umschließt der Patient mit seinem Mund das an dem Gehäuse 3 ausgebildete Mundstück 5 und kann das Aerosol inhalieren. Das Mundstück 5 kann mit dem Verschlussdeckel 26 abgedeckt werden.

In der Ausführungsform gemäß der Figur 1 sind die Kanäle 9a, 9b mit jeweils eigenen Düsenkörpern 8a, 8b versehen. Die Düsenkörper 8a, 8b weisen zum Durchtritt der jeweiligen Flüssigkeit an dem ersten Düsenkörper 8a zwei Düsenauslässe 11a₁, 11a₂ und an dem zweiten Düsenkörper 8b zwei weitere Düsenauslässe 11b₁, 11b₂ auf. Die Düsenauslässe 11b₁, 11b₂ des zweiten Düsenkörpers 8b sind derart ausgerichtet, dass sich die Flüssigkeitsstrahlen, die unter der Strahlrichtung 12 aus den Düsenauslässen 11b₁, 11b₂ austreten, im Impaktionsabstand 14 treffen. Gleiches gilt für die aus dem ersten Düsenkörper 8a aus den Düsenauslässen 11a₁, 11a₂ austretende Flüssigkeit. Es entstehen auf diese Weise zwei getrennte Aerosol-Wolken, die sich miteinander vermischen.

Die Düsenauslässe 11a₁, 11a₂ des ersten Düsenkörpers 8a sind ebenso wie die Düsenauslässe 11b₁, 11b₂ des zweiten Düsenkörpers 8b in der Entfernung 14a jeweils zueinander beabstandet.

Die Figur 2 zeigt eine Zerstäuber, bei der die Kanäle 9a, 9b im Bereich der Zerstäubungsvorrichtung 6 in einem gemeinsamen Düsenkörper 8 hineinlaufen und dort über einen getrennten einzelnen ersten Düsenauslaß 11 a und einen zweiten einzelnen Düsenauslaß 11 b austreten.

Wie in der Figur 2 zu sehen ist, treten die jeweiligen Flüssigkeiten unter einer Strahlrichtung 12 aus und treffen sich vor dem Zerstäuber 1. Die Flüssigkeiten treffen dabei unter dem Aufprallwinkel 13 zusammen. Durch das Aufeinandertreffen der Formulierungen wird eine besonders feine Vernebelung und Durchmischung der Formulierungen erzielt, wobei bei dieser Ausführungsform eine Aerosol-Wolke aus beiden Flüssigkeiten erzeugt wird.

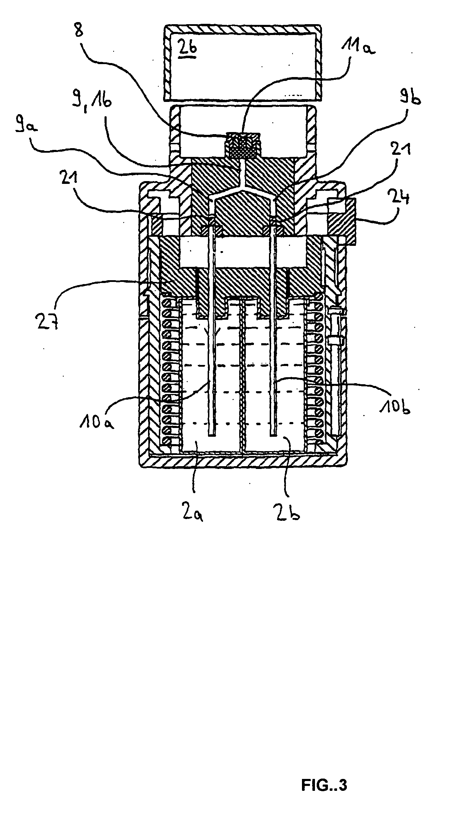

Die Figur 3 stellt einen dritten Zerstäuber dar, bei der der Kanal 9 als Mischkanal 16 ausgebildet ist, das heißt in dem Mischkanal 16 vereint sich der erste Kanal 9a und der zweite Kanal 9b. In dem Kanal 9a und dem Kanal 9b sind jeweils Hohlkolben 10a, 10b verschiebbar gelagert.

In der Figur 3 erfolgt ein Mischen der beiden Flüssigkeiten bereits beim Ansaugen, das heißt bei einer Abwärtsbewegung der beiden Hohlkolben 10a, 10b.

Bei einer Aufwärtsbewegung der beiden Hohlkolben 10a, 10b wird die jeweils zuvor angesaugte und in dem Kanal 9a, 9b befindliche Formulierung verdichtet und in Richtung des Düsenkörpers 8 ausgeschoben. In der Darstellung der Fig. 3 ist ein Düsenauslaß 11 a sichtbar. Eine Vermischung der über den Hohlkolben 10a aus der ersten Kartuschenkammer 2a und der über den Hohlkolben 10b aus der zweiten Kartuschenkammer 2b angesaugten Formulierung erfolgt bei dieser Ausführungsform also in dem Mischkanal 16. Ein Zurückströmen aus dem Mischkanal 16 zurück in die Kartuschenkammer wird durch die Ventile 21 verhindert.

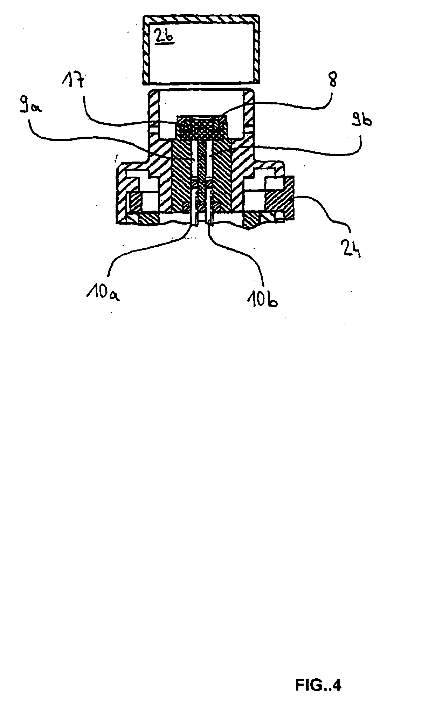

In der Figur 4 ist ein vierter Zerstäuber gezeigt, bei der anstelle des in Figur 3 zu erkennenden Mischkanals 16 eine in dem Düsenkörper 8 angeordnete Plenumkammer 17 ausgebildet ist. In dieser Plenumkammer 17 enden die Kanäle 9a, 9b, so dass dort bei einer Aufwärtsbewegung der Hohlkolben die Durchmischung der Formulierungen stattfindet. Abweichend von der Ausführungsform gemäß der Fig. 3 findet das Mischen nicht beim Ansaugen, sondern beim Ausbringen statt.

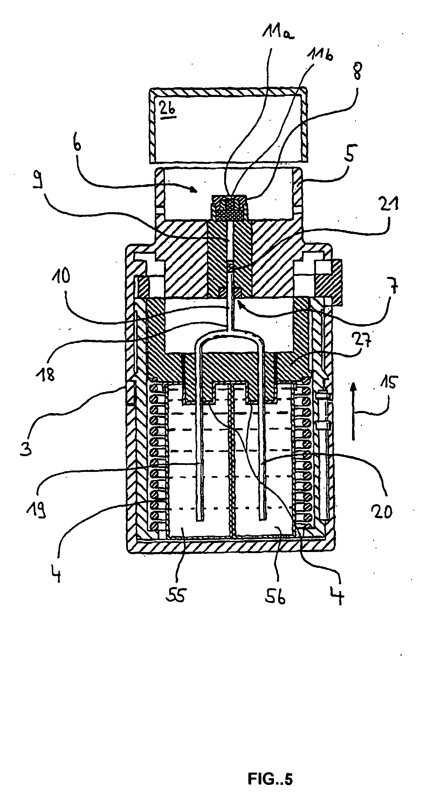

Die Figur 5 zeigt einen weiteren fünften Zerstäuber, bei der in einem Kanal 9 ein Hohlkolben 10 verschiebbar gelagert ist. Der einzige Kanal 9 endet in Strömungsrichtung 15 in dem Düsenkörper 8, durch welchen das Lösungsgemisch über die Düsenauslässe 11 a, 11 b austreten kann. Sichtbar ist in der Darstellung der Figur 5 nur der erste Düsenauslaß 11a. Außerhalb des Kolbens 9, im Bereich des kartuschenhalternahen Endes 18 des Hohlkolbens 10, zweigt sich der Hohlkolben 10 in einen ersten Teilkolben 19 und einen zweiten Teilkolben 20 auf. Dabei erstrecken sich beide Teilkolben 19, 20 in den Kartuschenhalter 4.

Abweichend von den zuvor dargestellten Ausführungsformen befinden sich in dem Kartuschenhalter 4 zwei körperlich getrennte Kartuschen, nämlich eine erste Kartusche 55 und eine zweite Kartusche 56. Diese Ausführungsform ist weniger bevorzugt.

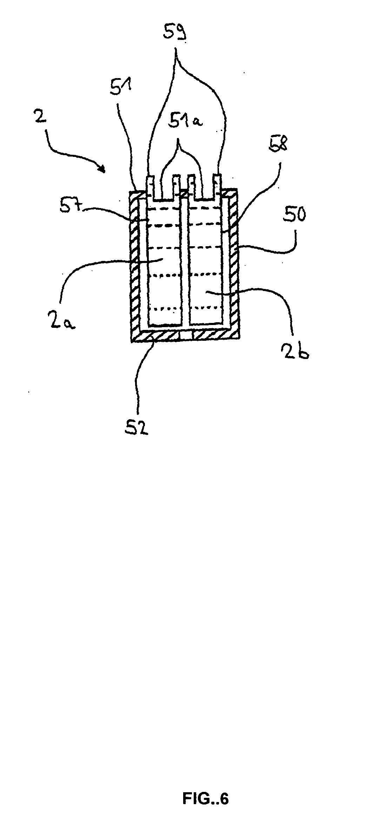

Die Fig. 6 zeigt eine erfindungsgemäße Kartusche 2 mit einer zylindrischen Kartuschenkammer 2a und einer daneben befindlichen ebenfalls zylindrischen Kartuschenkammer 2b.

Beide Kartuschenkammern 2a, 2b weisen eigene Innenwandungen 57, 58 auf, die beabstandet zu der Umfangswand 50 und der Bodenwand 52 angeordnet sind. Die Deckelwand 51 ist durch Einführhilfen 59 durchbrochen, in welche die Hohlkolben 10a, 10b eingreifen können und nach Durchstechen der Verschlussmembran 51 a in die erste Kartuschenkammer 2a und die zweite Kartuschenkammer 2b eingeschoben werden können.

### Bezugszeichenliste

- 1: Zerstäuber
- 2: Kartusche
- 2a: erste Kartuschenkammer
- 2b: zweite Kartuschenkammer
- 3: Gehäuse
- 4: Kartuschenhalter
- 5: Mundstück
- 6: Zerstäubungsvorrichtung
- 7: Verbindungsrohrsystem
- 8: Düsenkörper
- 8a: erster Düsenkörper
- 8b: zweiter Düsenkörper
- 9: Kanal
- 9a: erster Kanal
- 9b: zweiter Kanal
- 10: Hohlkolben
- 10a: erster Hohlkolben
- 10b: zweiter Hohlkolben
- 11 a: erster Düsenauslaß Düsenkörper
- 11 b: zweiter Düsenauslaß Düsenkörper
- 11a₁: erster Düsenauslaß erster Düsenkörper
- 11a₂: zweiter Düsenauslaß erster Düsenkörper
- 11b₁: erster Düsenauslaß zweiter Düsenkörper
- 11 b₂: zweiter Düsenauslaß zweiter Düsenkörper
- 12: Strahlrichtung
- 13: Aufprallwinkel
- 14: Impaktionsabstand
- 14a: Entfernung Düsenauslässe eines Düsenkörpers
- 15: Strömungsrichtung
- 16: Mischkanal
- 17: Plenumkammer
- 18: kartuschenhalternahes Ende des Hohlkolbens (10)
- 19: erster Teilkolben
- 20: zweiter Teilkolben
- 21a: erster Ventilkörper
- 21b: zweiter Ventilkörper
- 22: hinteres Ende Hohlkolben
- 23: Spannelement
- 24: Auslösetaste
- 25: axiale Richtung des Gehäuses (3)
- 26: Verschlussdeckel
- 27: Schubteller
- 50: Umfangswand Kartusche
- 51: Deckelwand Kartusche
- 51a: Verschlussmembran
- 52: Bodenwand Kartusche
- 55: erste Kartusche
- 56: zweite Kartusche
- 57: Innenwandung erste Kartusche
- 58: Innenwandung zweite Kartusche
- 59: Einführhilfen

## Patentansprüche

1. Zerstäuber zum Ausbringen von Flüssigkeiten für medizinische Zwecke aus mindestens einer in den Zerstäuber (1) einsetzbaren Kartusche (2, 55, 56), umfassend
- einen in dem Gehäuse (3) angeordneten Kartuschenhalter (4),
- ein an dem Gehäuse (3) ausgebildetes Mundstück (5), in welchem eine Zerstäubungsvorrichtung (6) angeordnet ist, und
- ein Verbindungsrohrsystem (7), welches die Zerstäubungsvorrichtung (6) und den Kartuschenhalter (4) verbindet, wobei die Zerstäubungsvorrichtung (6) mindestens einen ersten Düsenkörper (8) und das Verbindungsrohrsystem (7) mindestens einen ersten Kanal (9) mit einem darin verschiebbar gelagerten ersten Hohlkolben (10) aufweist, wobei der erste Hohlkolben (10) einen ersten Ventilkörper (21) umfaßt, wobei in dem ersten Kanal (9a) der erste Hohlkolben (10a) und in einem zweiten Kanal (9b) ein zweiter Hohlkolben (10b) verschiebbar gelagert sind, wobei der erste Hohlkolben (10a) und der zweite Hohlkolben (10b) in den Kartuschenhalter (4) hineinreicht
**dadurch gekennzeichnet, dass**
- die Zerstäubungsvorrichtung (6) einen ersten Düsenkörper (8a) mit zwei Düsenauslässen (11a₁, 11a₂) und einen zweiten Düsenkörper (8b) mit zwei Düsenauslässen (11b₁, 11b₂) umfasst, wobei der erste Düsenkörper (8a) mit dem ersten Kanal (9a) und der zweite Düsenkörper (8b) mit dem zweiten Kanal (9b) verbunden ist, und wobei die zwei Düsenauslässe (11a₁, 11a_{2;} 11b₁, 11b₂) eines Düsenkörpers (8a, 8b) zueinander geneigt sind.

## Claims

1. Nebuliser for delivering liquids for medical purposes from at least one cartridge (2, 55, 56) adapted to be inserted in the nebuliser (1), comprising
- a cartridge holder (4) arranged in the housing (3),
- a mouthpiece (5) configured on the housing (3), in which is disposed a nebuliser device (6) is located, and
- a connecting tube system (7) which connects the nebuliser device (6) and the cartridge holder (4), the nebuliser device (6) comprising at least one first nozzle body (8) and the connecting tube system (7) comprising at least one first channel (9) with a first hollow piston (10) slidably mounted therein, the first hollow piston (10) comprising a first valve body (21), the first hollow piston (10a) being slidably mounted in the first channel (9a) and a second hollow piston (10b) being slidably mounted in a second channel (9b), the first hollow piston (10a) and the second hollow piston (10b) extending into the cartridge holder (4),
**characterised in that**
- the nebuliser device (6) comprises a first nozzle body (8a) with two nozzle outlets (11a₁, 11a₂) and a second nozzle body (8b) with two nozzle outlets (11b_{1,} 11b₂), wherein the first nozzle body (8a) is connected to the first channel (9a) and the second nozzle body (8b) is connected to the second channel (9b), and wherein the two nozzle outlets (11a₁, 11a₂; 11b₁, 11b₂) of a nozzle body (8a, 8b) are inclined relative to each other.

## Revendications

1. Pulvérisateur servant à distribuer, à des fins médicales, des liquides provenant d'au moins une cartouche (2, 55, 56) pouvant être insérée dans le pulvérisateur (1), comportant :
- un élément de maintien de la cartouche (4) disposé dans le boîtier (3)
- un embout (5) réalisé au niveau du boîtier (3), dans lequel est disposé un dispositif de pulvérisation (6), et
- un système tubulaire de raccord (7), lequel relie le dispositif de pulvérisation (6) et l'élément de maintien de la cartouche (4), le dispositif de pulvérisation (6) présentant au moins un premier corps de buses (8) et le système tubulaire de raccord (7) présentant au moins un premier canal (9) muni d'un premier piston creux (10) logé de manière mobile à l'intérieur de ce dernier, le premier piston creux (10) comportant un premier corps de soupape (21), le premier piston creux (10a) étant logé de manière mobile dans le premier canal (9a) et un deuxième piston creux (10b) étant logé de manière mobile dans un deuxième canal (9b), le premier piston creux (10a) et le deuxième piston creux (10b) parvenant jusque dans l'élément de maintien de la cartouche (4),
**caractérisé en ce que**
- le dispositif de pulvérisation (6) comporte un premier corps de buses (8a) muni de deux sorties de buses (11a₁, 11a₂) et un deuxième corps de buses (8b) muni de deux sorties de buses (11b₁, 11b₂), le premier corps de buses (8a) étant relié au premier canal (9a) et le deuxième corps de buses (8b) étant relié au deuxième canal (9b), et les deux sorties de buses (11a₁, 11a₂, 11b₁, 11b₂) d'un corps de buses (8a, 8b) étant inclinées l'une par rapport à l'autre.
